# EUROPÄISCHE PATENTSCHRIFT

(11) **EP 0 483 242 B1**
(45) Veröffentlichungstag und Bekanntmachung des Hinweises auf die Patenterteilung: **03.05.1995**
(21) Anmeldenummer: 90911417.5
(22) Anmeldetag: 19.07.1990
(51) Int. Cl.: A61B 17/58

(54) **AUFNAHMETEIL FÜR EINE PEDIKELSCHRAUBE UND PEDIKELSCHRAUBE**
PEDICLE SCREW AND PEDICLE-SCREW HOLDER
ELEMENT DE RECEPTION D'UNE VIS DE PEDICULE, ET VIS DE PEDICULE

(30) Priorität: 20.07.1989 DE 3923996
(43) Veröffentlichungstag der Anmeldung: 06.05.1992
(73) Patentinhaber: Biedermann, Lutz, 78048 Villingen-Schwenningen (DE); Harms, Jürgen, Prof. Dr., 76337 Waldbronn (DE)
(72) Erfinder: Biedermann, Lutz, 78048 Villingen-Schwenningen (DE); Harms, Jürgen, Prof. Dr., 76337 Waldbronn (DE)
(74) Vertreter: Prüfer, Lutz H., Dipl.-Phys.
(86) Internationale Anmeldenummer: EP9001184
(87) Internationale Veröffentlichungsnummer: WO9101115

(56) Entgegenhaltungen:
- EP-A- 0 242 708
- DE-A- 3 711 013
- US-A- 4 805 602

## Beschreibung

Die Erfindung betrifft ein Aufnahmeteil für eine Pedikelschraube zum gelenkigen Verbinden einer einen Gewindeschaftteil und einen kugelförmigen bzw. kugelsegmentförmigen Kopf aufweisenden Schraube mit einer Stange sowie eine Pedikelschraube zur Stabilisierung von Wirbelsäulensegmenten.

Aus der DE-AS 26 49 042 ist eine Pedikelschraube bekannt mit einem Gewindeteil und einem starr damit am kopfseitigen Ende vorgesehenen Aufnahmeteil. Es werden mehrere Paare solcher Schrauben jeweils in einem Abstand zueinander beidseitig von der Wirbelsäule in die Wirbelkörper eingeschraubt. Die jeweiligen Aufnahmeteile weisen Aufnahmeschlitze auf. Durch diese Aufnahmeschlitze der rechten bzw. linken Gruppe der Schrauben wird jeweils eine Gewindestange geführt. Mit Hilfe von Fixierungsschrauben wird die Stange dann am jeweiligen Aufnahmeteil fixiert. Ein Nachteil dieser Lösung besteht darin, daß es sehr schwierig ist, die Schrauben einerseits fest in die Wirbelkörper einzuschrauben und andererseits die Schrauben in zwei Ebenen gerade so zu steilen, daß die Achsen der Aufnahmeschlitze in den übereinander befindlichen Aufnahmeteilen so ausgerichtet sind, daß die Gewindestange ohne Verspannung der Schrauben durch die Aufnahmeschlitze hindurchführbar ist. Schon der Versuch erfordert sehr viel Zeit, was bei einer Operation an der Wirbelsäule ein großer Nachteil ist. Darüber hinaus läßt sich eine so genaue Ausrichtung fast nicht erreichen. Das Ergebnis ist, daß erhebiche Scherkräfte auf die Gewindestangen ausgeübt werden, was dazu führt, daß in der späteren Benutzung nach Abschluß der Operation die Stangen sogar abbrechen können.

Aus der EP 0 242 708 A ist eine Pedikelschraube bekannt aus einer eigentlichen Schraube mit einem Gewindeschaftteil und einem kugelförmigen bzw. kugelsegmentförmigen Kopf und einem gelenkig damit verbindbaren Aufnahmeteil. Das Aufnahmeteil weist zwei durch einen Ring zusammengehaltene Kopfhälften auf, die auf ihren einander zugewandten Innenseiten hohlkugelsegmentförmige Abschnitte aufweisen, die den Kopf im zusammengesetzten Zustand so umfassen, daß dieser in der so gebildeten Hohlkugel um deren Mittelpunkt schwenkbar gehalten wird. Damit wird eine an sich sehr gut funktionierende Pedikelschraube geschaffen. Bei der Operation werden dann aber Schrauben verschiedenster Längen und ggf. auch verschiedenster Durchmesser benötigt. Bei der bisher bekannten Schraube ist es erforderlich, die kompletten Schrauben für die gewünschten Größen vorrätig zu halten. Wegen der erforderlichen hohen Präzision der beiden die kugelsegmentförmigen Abschnitte aufweisenden Schalenhälften und des diese zusammenhaltenden Ringes sind die Lagerkosten für solche kompletten Schrauben sehr hoch.

Aus der US-A-4,805,602 ist eine Pedikelschraube mit einem einstückigen Aufnahmeteil bekannt, bei der die Schraube mit der Schaftseite durch das Aufnahmeteil eingeführt werden kann. Bei dieser Vorrichtung besteht jedoch keine Möglichkeit, die Schraube gegen Lockern bzw. Verdrehen zu sichern.

Aufgabe der Erfindung ist es, eine Möglichkeit zu schaffen, mit der die Kosten für die Vorratshaltung solcher Schrauben gesenkt werden.

Diese Aufgabe wird durch ein Aufnahmeteil nach Patentanspruch 1 gelöst.

Eine mit einem solchen Aufnahmeteil gebildete Pedikelschraube ist in Patentanspruch 2 gekennzeichnet.

Weiterbildungen der Erfindung sind in den Unteransprüchen gekennzeichnet.

Durch die Erfindung wird erreicht, daß zur Bevorratung nur die Schraubenschäfte mit unterschiedlicher Länge und unterschiedlicher Dicke, aber mit einheitlichem Kopf bevorratet werden. Die Lagerkosten werden dadurch erheblich gesenkt.

Weitere Merkmale und Zweckmäßigkeiten der Erfindung ergeben sich aus der Beschreibung eines Ausführungsbeispieles anhand der Figuren. Von den Figuren zeigen:
- Fig. 1: eine explosionsartige Seitenansicht der Pedikelschraube mit dem Aufnahmeteil, teilweise in geschnittener Darstellung, und
- Fig. 2: eine Schnittdarstellung durch das Aufnahmeteil mit eingesetzter Schraube und mit mit dem Aufnahmeteil verbundener Stange, in vergrößerter Darstellung.
- Fig. 3: eine gegenüber der in Fig. 1 um 90° um die Symmetrieachse gedrehte Darstellung in vergrößertem Maßstab.

Die Pedikelschraube 1 weist eine eigentliche Schraube 2 mit Gewindeschaftteil 3 und einstückig damit ausgebildetem kugelsegmentförmigem Kopf 4 sowie den Aufnahmeteil 5 auf.

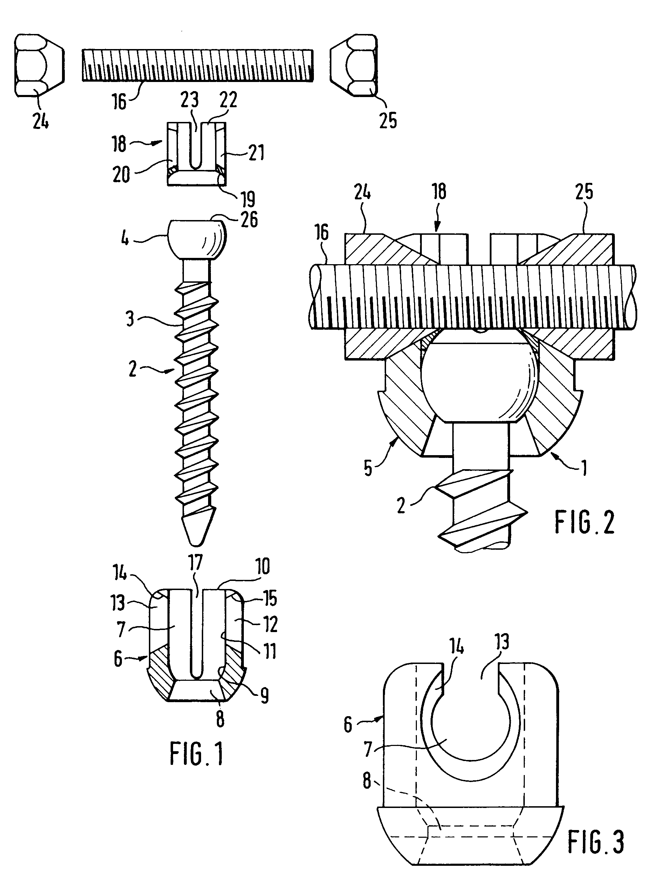

Der Aufnahmeteil 5 weist ein Gehäuse 6 zum Aufnehmen der Schraube 2 auf. Das Gehäuse weist im Inneren einen Aufnahmeraum 7 auf. Dieser ist in axialer Richtung des Aufnahmeraumes gesehen auf seinem einen Ende von einer Bohrung 8 begrenzt, deren Durchmesser kleiner ist als der zweifache Radius des kugelsegmentförmigen Abschnittes des Kopfes 4. Unmittelbar im Inneren an die Bohrung angrenzend ist ein hohlkugelsegmentförmiger Abschnitt 9 vorgesehen, dessen Radius im wesentlichen gleich dem Radius des kugelsegmentförmigen Abschnittes des Kopfes 4 ist. Der hohlkugelsegmentförmige Abschnitt 9 geht unmittelbar über in einen sich bis zu einer der Bohrung 8 gegenüberliegenden Öffnung 10 erstreckenden hohlzylindrischen Abschnitt 11, dessen Durchmesser gerade so wenig größer als der zweifache Radius des kugelsegmentförmigen Abschnittes des Kopfes 4 ist, daß der Kopf bei Einführen der Schraube 2 in den Aufnahmeteil in die in Fig. 2 gezeigte Position einführbar ist, in der der Kopf am hohlkugelsegmentförmigen Abschnitt 9 anliegt. Die Bohrung 8 ist, wie insbesondere aus Fig. 2 ersichtlich ist, koaxial zur Achse des zylinderförmigen Abschnittes ausgerichtet und weist von innen nach außen gesehen die Form eines Kegelabschnittes mit nach außen divergierender Wandung auf. Dadurch ist es möglich, die eingesetzte Schraube innerhalb eines durch den Durchmesser diesem Kegelabschnittes bestimmten Winkelbereiches um die Achse des hohlzylindrischen Abschnittes zu schwenken.

In einem Abstand von dem hohlkugelsegmentförmigen Abschnitt 9 weist das Gehäuse zwei um 180° gegeneinander versetzte und sich parallel zur Achse des hohlzylindrischen Abschnittes 11 erstreckende und zur Öffnung 10 hin offene Aufnahmeschlitze 12, 13 auf. Jeder der Aufnahmeschlitze weist wie bei der in der genannten EP 0 242 708 A beschriebenen Pedikelschraube auf der Außenseite jeweils eine Versenkunmg 14, 15 auf. Die Breite der Aufnahmeschlitze ist so gewählt, daß ein aufzunehmende Gewindestange 16 lose durch diese hindurchführbar ist. Wie am besten aus Fig. 1 ersichtlich ist, weist das Gehäuse ferner zwei um die Symmetrieachse des Gehäuses um 90° gegen die Aufnähmeschlitze 12, 13 versetzte Schlitze 17 auf, die sich von der Öffnung 10 bis etwa zum inneren Rand des Aufnahmeraumes 7 erstrecken.

Ferner ist ein Druckelement 18 in Form eines zylinderförmigen Einsatzes vorgesehen. Der Außendurchmesser des Zylinders ist im wesentlichen gleich dem Innendurchmesser des hohlzylinderförmigen Abschnittes 11 und gerade um so viel kleiner gewählt, daß das Druckelement in dem Hohlzylinder verschiebbar ist. Wie am besten aus Fig. 1 ersichtlich ist, ist das Druckelement bevorzugt als Hohlzylinder ausgebildet. Das beim Einsetzen in das Gehäuse 6 dem hohlkugelsegmentförmigen Abschnitt zugewandte Ende weist einen ebenfalls hohlkugelsegmentförmigen Abschnitt 19 auf. Der Radius der Krümmung ist im wesentlichen gleich dem Radius der Krümmung des hohlkugelsegmentförmigen Abschnittes 9 und im wesentlichen gleich dem Radius des kugelsegmentförmigen Abschnittes 4 gewählt.

Das Druckelement 18 weist ferner zwei den Aufnahmeschlitzen 12, 13 entsprechende Aufnahmeschlitze 20, 21 auf, die sich in einem Abstand von dem hohlkugelsegmentförmigen Abschnitt 19 parallel zur Symmetrieachse des Druckelementes und bis zu der gegenüberliegenden Öffnung 22 des Druckelementes erstrecken und die zur Aufnahme der Gewindestange 16 bestimmt sind. Ferner sind wiederum im wesentlichen um 90° um die Symmetrieachse gegen die Aufnahmeschlitze versetzt zwei gegenüberliegende Schlitze 23 vorgesehen, die unmittelbar oberhalb des hohlkugelsegmentförmigen Abschnittes 19 beginnen und auf der gegenüberliegenden Seite offen sind.

Ferner sind Muttern 24, 25 vorgesehen, die dazu dienen, in der in Fig. 2 gezeigten Weise die Stange 16 mit der Pedikelschraube zu verbinden.

Grundsätzlich ist es möglich, den Kopf 4 auf seiner dem Gewindeschaftteil 3 abgewandten Seite kugelförmig auszubilden. Die Aufnahmeschlitze und die Versenkungen in dem Gehäuse 6 sind dann so zu wählen, daß die Stange 16 in einem ausreichenden Abstand über dem Kopf liegt, damit dieser beim Verschwenken der Schraube nicht mit der Stange bzw. den diese haltenden Schrauben in Kontakt gelangt. Bevorzugt weist der Kopf auf seinem dem Gewindeschaftteil 3 abgewandten Ende eine sich senkrecht zur Schraubenachse erstreckende ebene Fläche 26 auf. In diese ist eine Vertiefung eingelassen, in die mit einem Schraubendreher Werkzeug zum Drehen der Schraube eingegriffen werden kann.

Vor dem Einsetzen der Pedikelschraube wird zunächst die Schraube 2 mit gewünschtem Schaftdurchmesser und gewünschter Schaftlänge in die in Fig. 2 gezeigte Position so eingesetzt, daß der Kopf mit seinem kugelsegmentförmigen Teil in dem hohlkugelsegmentförmigen Abschnitt ruht. Dann wird die Schraube in den jeweiligen Wirbelkörper eingeschraubt, in dem mit dem zugehörigen Werkzeug in die dafür vorgesehene Vertiefung des Kopfes 4 eingegriffen wird. Nach dem Einschrauben (gewünschtenfalls auch vorher) wird das Druckelement in der am besten aus Fig. 2 ersichtlichen Weise so in den hohlzylinderförmigen Abschnitt 11 eingesetzt, daß sein hohlkugelsegmentförmiger Abschnitt 19 auf dem sphärischen Bereich des Kopfes 4 ruht. Die Aufnahmeschlitze von Gehäuse 6 und Druckelement 18 werden parallel zueinander ausgerichtet. Dann wird die damit zu verbindende Stange 16 in die Aufnahmeschlitze eingelegt und durch Anziehen der Muttern 24 und 25 fest damit verbunden. Der Grund der Aufnahmeschlitze 20, 21 ist um so viel zum freien Ende der Schlitze hin versetzt, daß beim Verschrauben mit den die Stange haltenden Muttern eine zusätzliche Schubkraft in Richtung zu dem hohlkugelsegmentförmigen Abschnitt hin auf den Kopf 4 wirkt. Durch die Schlitze 17 und 23 ist ein geringes Nachgeben der Zylinderwandung des Gehäuses 6 dahingehend möglich, daß eine noch stärkere Klemmkraft auf den Kopf wirkt. Durch das Vorsehen des Druckelementes 18 wird erreicht, daß bei diesem für den Halt erforderlichen Zusammendrücken der Kopf nicht etwa von dem innigen Kontakt mit dem hohkugelsegmentförmigen Abschnitt 19 in das Innere hineingedrückt, sondern vollständig von einem hohlkugelförmigen Bereich umgeben von dem Druckelement sogar in Richtung des hohlkugelsegmentförmigen Abschnittes 19 gedrückt wird. Dadurch wird eine feste Arretierung in der eingestellten Winkelposition erreicht.

## Patentansprüche

1. Pedikelschraube (2) mit einem Aufnahmeteil (5) zum gelenkigen Verbinden der einen Gewindeschaftteil (3) und einen kugelförmigen bzw. kugelsegmentförmigen Kopf (4) aufweisenden Schraube (2) mit einer Stange (16), wobei eine Vorrichtung zur Sicherung der Schraube (2) gegen Verdrehen vorgesehen ist und wobei das Aufnahmeteil (5) einen Aufnahmeraum (7) aufweist, der an seinem einen Ende eine Bohrung (8) zum Hindurchführen des Gewindeschaftteiles (3) und angrenzend an diesen einen hohlkugelsegmentförmigen Abschnitt (9) zum Anliegen des Kopfes (4) der aufzunehmenden Schraube (2) besitzt,
dadurch gekennzeichnet, daß das Aufnahmeteil (5) einstückig ausgebildet ist und auf der der Bohrung (8) gegenüberliegenden Seite eine Öffnung (10) zum Einführen der Schraube (2) aufweist, und daß ein Druckelement (18) vorgesehen ist, welches durch die Öffnung (10) einführbar ist und in zusammengesetztem Zustand eine Kraft so auf den Kopf (4) ausübt, daß dieser gegen den hohlkugelsegmentförmigen Abschnitt (9) gedrückt wird.

2. Pedikelschraube nach Anspruch 1,
dadurch gekennzeichnet, daß das Aufnahmeteil (5) an seinem der Bohrung (8) abgewandten Abschnitt wenigstens einen sich bis zum offenen Rand hin erstreckenden Schlitz (17) und winkelmäßig gegen diesen versetzt zwei einander gegenüberliegende Schlitze (12, 13) zum Hindurchführen der Stange (16) und Arretieren derselben mittels Muttern (24, 25) aufweist.

3. Pedikelschraube nach Anspruch 1,
dadurch gekennzeichnet, daß das Druckelement (18) auf seinem dem Kopf (4) zugewandten Ende einen zu dem Kopf hin gerichteten hohlkugelsegmentförmigen Abschnitt (19) aufweist.

4. Pedikelschraube nach einem der Ansprüche 1 bis 3,
dadurch gekennzeichnet, daß die Radien der hohlkugelsegmentförmigen Abschnitte (9, 19) im wesentlichen gleich sind.

5. Pedikelschraube nach Anspruch 3,
dadurch gekennzeichnet, daß das Druckelement (18) einen zylinderförmigen Abschnitt aufweist, dessen Außendurchmesser im wesentlichen gleich dem Innendurchmesser des Aufnahmeraums (7) ist.

6. Pedikelschraube nach einem der Ansprüche 3 bis 5,
dadurch gekennzeichnet, daß das Druckelement (18) zwei den Schlitzen (12, 13) zum Aufnehmen der Gewindestange (16) entsprechende Schlitze (20, 21) aufweist, deren Grund sich soweit von dem mit dem Kopf (4) in Eingriff bringbaren Rand weg erstreckt, daß beim Verschrauben mit den die Stange (16) haltenden Muttern (24, 25) eine Schubkraft in Richtung zu dem hohlkugelsegmentförmigen Abschnitt (9) hin auf den Kopf (4) wirkt.

## Claims

1. Pedicle screw (2) with a holder part (5) for hinged connection of the screw (2), which has a threaded shank part (3) and a spherical or spherical-segment-shaped head (4), to a rod (16), a device being provided for securing the screw (2) against twisting, and the holder part (5) having a holder area (7) which at its one end has a bore (8) for the threaded shank part (3) to be guided through, and adjoining this bore a section (9) which is in the shape of a hollow spherical segment and on which there bears the head (4) of the screw (2) to be held; characterized in that the holder part (5) is designed in one piece and has, on the side opposite the bore (8), an opening (10) for the introduction of the screw (2); and in that a pressure element (18) is provided which can be introduced through the opening (10) and, in the assembled state, exerts a force on the head (4) such that the latter is pressed against the section (9) in the shape of a hollow spherical segment.

2. Pedicle screw according to Claim 1, characterized in that the holder part (5) has, on its section facing away from the bore (8), at least one slot (17) which extends up to the open edge, and, angularly offset in relation to this slot (17), two mutually opposite slots (12, 13) for the rod (16) to be guided through and for the latter to be locked by means of nuts (24, 25).

3. Pedicle screw according to Claim 1, characterized in that the pressure element (18) has, at its end facing towards the head (4), a section (19) in the shape of a hollow spherical segment directed towards the head.

4. Pedicle screw according to one of Claims 1 to 3, characterized in that the radii of the sections (9, 19) in the shape of hollow spherical segments are essentially equal.

5. Pedicle screw according to Claim 3, characterized in that the pressure element (18) has a cylindrical section whose external diameter is essentially equal to the internal diameter of the holder area (7).

6. Pedicle screw according to one of Claims 3 to 5, characterized in that the pressure element (18) has two slots (20, 21) corresponding to the slots (12, 13) for holding the threaded rod (16), the base of which slots (20, 21) extends so far from the edge which can be brought into engagement with the head (4) that, upon screwing with the nuts (24, 25) holding the rod (16), a shearing force in the direction of the section (9) in the shape of a hollow spherical segment acts upon the head (4).

## Revendications

1. Vis à pédicule (2) comprenant un élément de réception (5) pour l'assemblage articulé de la vis (2) présentant une partie de tige filetée (3) et une tête ayant une forme sphérique ou de segment sphérique (4) avec une tige (16), un dispositif étant prévu pour protéger la vis (2) contre la torsion et l'élément de réception (5) présentant un espace de réception (7) qui possède à l'une de ses extrémités un alésage (8) pour le passage de la partie de tige filetée (3) et, contigue à celle-ci, une section (9) en forme de segment sphérique creux pour l'appui de la tête (4) de la vis (2) à loger, caractérisée en ce que l'élément de réception (5) est constitué d'un seul bloc et présente une ouverture (10) pour l'introduction de la vis (2) sur le côté opposé à l'alésage (8), et en ce qu'on prévoit un élément de pression (18) qui peut être introduit par l'ouverture (10) et exerce une force à l'état monté sur la tête de façon que celle-ci soit appuyée contre la section (9) en forme de segment sphérique creux.

2. Vis à pédicule selon la revendication 1, caractérisée en ce que l'élément de réception (5) présente sur sa portion opposée à l'alésage (8) au moins une fente (17) s'étendant jusqu'au bord ouvert et, de façon décalée en angle par rapport à celle-ci, deux fentes (12, 13) opposées pour le passage de la tige (16) et le blocage de celle-ci au moyen d'écrous (24, 25).

3. Vis à pédicule selon la revendication 1, caractérisée en ce que l'élément de pression (18) présente une section (19) en forme de segment sphérique creux et dirigée vers la tête sur son extrémité opposée à la tête (4).

4. Vis à pédicule selon l'une des revendications 1 à 3, caractérisée en ce que les rayons des sections (9, 19) en forme de segment sphérique creux sont sensiblement identiques.

5. Vis à pédicule selon la revendication 3, caractérisée en ce que l'élément de pression (18) présente une portion de forme cylindrique dont le diamètre extérieur est sensiblement égal au diamètre intérieur de l'espace de réception (7).

6. Vis à pédicule selon l'une des revendications 3 à 5, caractérisée en ce que l'élément de pression (18) présente deux fentes (20, 21) correspondant aux fentes (12, 13) pour le logement de la tige filetée (16), dont le fond s'étend en partant du bord qui peut être mis en contact avec la tête (4) dans la mesure où, lors du vissage avec les écrous (24, 25) maintenant la tige (16), une force de poussée agit sur la tête (4) en direction de la section (9) en forme de segment sphérique creux.
